# EUROPÄISCHE PATENTSCHRIFT

(11) **EP 1 586 342 B1**
(45) Veröffentlichungstag und Bekanntmachung des Hinweises auf die Patenterteilung: **03.01.2007**
(21) Anmeldenummer: 05015620.7
(22) Anmeldetag: 07.05.1999
(51) Int. Cl.: A61M 5/32, A61M 5/20, A61M 5/24

(54) **Vorrichtung zur Verabreichung eines injizierbaren Produkts**
Device for administering an injectable product
Dispositif d'administration d'un produit injectable

(30) Priorität: 15.05.1998 DE 19821933
(43) Veröffentlichungstag der Anmeldung: 19.10.2005
(62) Teilanmeldung aus: 99810406.1
(73) Patentinhaber: TecPharma Licensing AG, 3401 Burgdorf (CH)
(72) Erfinder: Kirchhofer, Fritz, 3454 Sumiswald (CH); Steck, Jürg, 3422 Kirchberg (CH); Hostettler, Peter, 3423 Ersigen (CH); Jost, Stephan, 3203 Mühleberg (CH); Heiniger, Hanspeter, 4932 Lotzwil (CH)
(74) Vertreter: Wess, Wolfgang

(56) Entgegenhaltungen:
- EP-A- 0 518 416
- WO-A-95/35126
- US-A- 5 219 338
- US-A- 5 658 259

## Beschreibung

Die Erfindung betrifft Vorrichtungen zur Verabreichung injizierbarer Produkte, insbesondere zur Injektion von medizinisch oder kosmetisch wirksamen Produkten.

Vorrichtungen, wie die Erfindung sie unter anderem insbesondere auch betrifft, sind Injektionspens. Im allgemeinen weist ein Injektionspen ein langgestrecktes hohlzylindrisches Gehäuse auf, in dem ein mit dem zu verabreichenden Produkt gefülltes Behältnis in Form einer Ampulle mit daran befestigter, in Längsrichtung des Gehäuses weisender Injektionsnadel und eine Antriebseinrichtung für einen im Behältnis aufgenommenen Kolben angeordnet sind. Unter der Einwirkung eines Abtriebsglieds der Antriebseinrichtung wird der Kolben im Behältnis in eine Vorschubrichtung vorgeschoben, wodurch eine vorgegebene Produktdosis ausgeschüttet wird. Zum Vorschieben des Kolbens wird das Abtriebsglied seinerseits manuell oder durch die Antriebskraft eines in der Antriebseinrichtung vorgesehenen Antriebselements in Vorschubrichtung relativ zum Gehäuse vorgeschoben. Im letzteren Fall ist in der Antriebseinrichtung Energie gespeichert, die durch Auslösen der Antriebseinrichtung in die das Abtriebsglied vortreibende Antriebskraft umgewandelt wird.

Im Verlaufe des Vorschiebens des Abtriebsglieds wird die gespeicherte Energie oder zumindest ein Teil davon aufgezehrt. Die vom Antriebselement auf das Abtriebsglied ausgeübte Antriebskraft nimmt im Verlaufe des Vorschiebens des Antriebsglieds infolgedessen ab. Die Vorschubgeschwindigkeit des Abtriebsglieds und dadurch die Ausschüttrate nehmen zum Ende der Ausschüttung hin ab, d.h. die Ausschüttung erfolgt über ein gesamtes Ausschüttungsintervall betrachtet ungleichmäßig. Weitere Störeinflüsse werden insbesondere durch Ungleichmäßigkeiten des Innendurchmessers der Ampulle hervorgerufen, da hierdurch die auf den Kolben wirkenden Wandreibungskräfte über den Verschiebeweg des Kolbens gesehen nicht konstant sind.

Aus dem Stand der Technik ist die Patentschrift WO-A-95/35126 bekannt. Diese Patentschrift beschreibt eine Injektionsvorrichtung mit einer im Innern dieser Vorrichtung sich befindenden Nadel, die durch eine flexible Hülle geschützt ist. Diese flexible Hülle wird dabei für die Injektion durch einen Rückstreifer, der fest mit dem Basisteil der Injektionsvorrichtung verbunden ist, in Richtung des Nadelschafts, also in proximale Richtung, zurückgeschoben. Am Ende der Injektion kehrt der Rückstreifer wieder in die Ausgangslage zurück. Der Rückstreifer ist fest am Basisteil jedoch relativ zur Nadel hin- und herschiebbar angebracht.

Die Erfindung hat es sich zur Aufgabe gemacht, bei Vorrichtungen zur Verabreichung injizierbarer Produkte eine gleichmäßige Produktausschüttung sicherzustellen.

Nach der Erfindung umfaßt eine Vorrichtung zur Verabreichung eines injizierbaren Produkts wenigstens ein Basisteil, ein vom Basisteil aufgenommenes Behältnis, aus dem durch Verschieben eines darin aufgenommenen Kolbens in eine Vorschubrichtung Produkt durch eine Nadel hindurch ausgeschüttet wird, und eine Antriebseinrichtung, die wenigstens ein Abtriebsglied und ein Antriebselement aufweist, wobei das Antriebselement auf das Abtriebsglied zumindest im Falle einer Betätigung der Antriebseinrichtung eine Antriebskraft ausübt, durch die das Abtriebsglied in Vorschubrichtung des Kolbens verschoben wird und dabei den Kolben im Behältnis vorschiebt. Die Antriebskraft kann eine Federkraft sein, sie kann beispielsweise aber auch durch ein Druckfluid, vorzugsweise Druckluft, auf das Abtriebsglied ausgeübt werden. Die Betätigung der Antriebseinrichtung besteht darin, daß eine das Vorschieben des Abtriebsglieds verhindernde Blockierung gelöst wird, gegebenenfalls wird dabei auch erst die Antriebskraft in Einwirkung auf das Abtriebsglied gebracht. Nach der Erfindung weist die Vorrichtung Mittel zur Erzeugung einer kontrollierten Dämpfungskraft auf, die der Antriebskraft im Verlaufe eines Vorschiebens des Kolbens zusätzlich zu allen nicht vermeidbaren Gegenkräften entgegenwirkt. Die nicht vermeidbaren Gegenkräfte sind im wesentlichen die beim Vorschieben des Kolbens auf den Kolben ausgeübten Reibungskräfte und von der Verdrängungsarbeit des Kolbens herrührende Kräfte. Indem zusätzlich zu diesen beim Vorschieben zwangsläufig auftretenden Gegen-kräften eine Dämpfungskraft der Antriebskraft entgegengesetzt wird, kann die auf das Abtriebsglied wirkende resultierende Differenzkraft aus der erzeugten Antriebskraft und allen nicht vermeidbaren Gegenkräften sowie der erfindungsgemäß zusätzlich erzeugten Dämpfungskraft wesentlich genauer kontrolliert werden, als dies bei den bekannten Vorrichtungen der Fall ist.

Die Dämpfungskraft wird vorteilhafterweise so eingestellt, daß das Produkt mit möglichst konstanter Ausschüttrate verabreicht wird. In den meisten Anwendungsfällen ist die Ausschüttrate konstant, wenn der Kolben mit konstanter Geschwindigkeit vorgeschoben wird. Dementsprechend wird die Dämpfungskraft in einer Ausführungsform in Abhängigkeit von der Vorschubgeschwindigkeit des Kolbens, vorzugsweise in Abhängigkeit unmittelbar von der Vorschubgeschwindigkeit des Abtriebsglieds erzeugt. Eine Beschleunigung erhöht und eine Verzögerung verringert die Dämpfungskraft; bei konstanter Vorschubgeschwindigkeit bleibt sie konstant. Besonders bevorzugt wird eine Selbstregelung.

In bevorzugten Ausführungsbeispielen nimmt die Dämpfungskraft im Verlaufe der Vorschubbewegung wenigstens einmal ab. Vorzugsweise ist der über die Verschiebestrecke des Kolbens aufgetragene Verlauf der Dämpfungskraft dem Verlauf der vom Antriebselement ausgeübten Antriebskraft angepaßt. Bei Energieverzehr im Antriebselement nimmt die Dämpfungskraft in Anpassung an die Abnahme der Antriebskraft ab.

In einer ersten Ausführungsvariante wird die Dämpfungskraft dadurch erzeugt, daß zum Vorschieben des Kolbens eine Volumenänderungsarbeit aufgrund einer sich beim Vorschieben des Kolbens vergrößernden oder verkleinernden Kammer zu leisten ist, in der ein Druckausgleich nur verzögert stattfindet. Dieses System ist selbstregelnd, da eine Geschwindigkeitsänderung eine vorzeichengleiche Änderung der Dämpfungskraft bewirkt.

In einer zweiten Ausführungsvariante ist die Dämpfungskraft selbst eine Reibungskraft. Durch die konstruktive Gestaltung der hierfür in Reibeingriff stehenden Komponenten wird die in diesem Fall auf Reibung beruhende Dämpfungskraft ebenfalls kontrolliert.

Vorzugsweise findet die Erfindung bei Injektionsgeräten Verwendung. Sie ist auf diese Verwendung jedoch nicht beschränkt. Grundsätzlich ist sie bei allen Vorrichtungen zur Produktverabreichung gewinnbringend einsetzbar, bei denen die Antriebskraft ein Vorschieben des Abtriebsglieds unmittelbar bewirkt und/oder die Vorschubbewegung des Kolbens erst aus dem Zusammenspiel der Antriebskraft mit nicht vermeidbaren, nur innerhalb von Fertigungstoleranzen vorgebbaren Gegenkräften ergibt.

Eine weitere Aufgabe der Erfindung besteht darin, das Entfernen einer auf die Nadel gesteckten Nadelschutzkappe bei solchen Vorrichtungen zu verbessern, bei denen die Nadel zum Transport der Vorrichtung von einer Nadelschutzhülse umgeben wird und lediglich bei der Injektion über ein vorderes Ende der Nadelschutzhülse hinaus vorragt. Bei bekannten Vorrichtungen dieser Art, insbesondere halbautomatischen Injektionsgeräten und vollautomatischen Injektionsgeräten, sogenannten Autoinjektoren, ist die Nadelschutzhülse geschlitzt, damit der Verwender schlitzdurchgreifend die Nadelschutzkappe entfernen kann. Dies bedeutet jedoch, daß die Nadel selbst bereits in der Transportstellung der Vorrichtung und insbesondere beim Ansetzen der Vorrichtung zum Zwecke des Einstechens der Nadel sichtbar ist, wodurch beim Verwender eine psychologische Barriere gegen das Einstechen der Nadel aufgebaut werden kann.

Die Erfindung löst die Aufgabe dadurch, daß ein Abstreifer mit der Vorrichtung verbunden ist, derart, daß der Abstreifer gegen die Vorschubrichtung der Nadel verschiebbar ist. Nachdem der Abstreifer seine Funktion erfüllt hat, d.h. die Nadelschutzkappe von der Nadel abgestreift hat, so daß sie auf einfache Weise gänzlich von der Vorrichtung entfernt werden kann, behindert der erfindungsgemäße Abstreifer bei der Injektion nicht, obgleich er noch immer mit der Vorrichtung verbunden ist, da er beim Einstechen der Nadel entweder in die Nadelschutzhülse hinein oder über sie geschoben wird und dann die Nadel auch über ein vorderes Ende des Abstreifers vorragt. Dabei ist der Abstreifer mit Eingriffsmitteln zum Klemmen oder Greifen der Nadelschutzkappe versehen, die jedoch ein Vorwärtsschieben der Nadel relativ zum Abstreifer und zur Nadelschutzhülse nicht behindern, wenn die Nadelschutzkappe entfernt worden ist.

Ferner hat sich die Erfindung zur Aufgabe gemacht, eine Vorrichtung, wie die Erfindung sie betrifft, so weiterzubilden, daß sie auch nach der Verabreichung des Produkts sicher zu handhaben ist. Ein Sicherheitsproblem stellt eine offen vorstehende Nadel nach der Verabreichung einer Produktdosis dar.

Die Erfindung löst die Aufgabe dadurch, daß eine mit der Vorrichtung in und gegen die Vorschubrichtung der Nadel verschiebbar verbundene Nadelschutzhülse in einer Ausgangsstellung, in der sie die Nadel bis über deren Spitze hinaus schützend umgibt, gegen ein Zurückschieben entgegen der Vorschubrichtung der Nadel blockierbar ist. Vorzugsweise erfolgt das Blockieren durch Anbringen eines Sperrelements oder, besonders bevorzugt, automatisch im Verlaufe des Herausziehens der eingestochenen Nadel nach der Verabreichung.

Desweiteren soll die Betriebssicherheit einer Vorrichtung, wie die Erfindung sie betrifft, und die in Weiterbildung ein Autoinjektionsgerät ist, bei dem die Nadel automatisch durch Vorschieben des Behältnisses relativ zum Basisteil vorschiebt, verbessert werden. Bei solchen Vorrichtungen wird das Behältnis zum Einstechen der Nadel gegen eine elastische Rückstellkraft vorgeschoben, die das Behältnis zum Austauschen in seine hintere Position zurückschiebt.

Die Erfindung setzt bei der Erkenntnis an, daß beim Abziehen einer über die Nadel gesteckten Nadelschutzkappe das Behältnis gegen die elastische Rückstellkraft ein Stück weit vorgezogen und beim Abziehen der Nadelschutzkappe aufgrund der Rückstellkraft abrupt in die hintere Position zurückschnellt und dabei beschädigt werden kann. Die Betriebssicherheit wird nicht nur durch die Gefahr einer Beschädigung des Behältnisses gefährdet, sondern auch durch möglicherweise ausbrechende Behältnissplitter, die eine zum Zwecke des Einstechens der Nadel erforderliche Vorschubbewegung des Behältnisses blockieren können. Dieser Gefahr wird erfindungsgemäß dadurch vorgebeugt, daß das Behältnis in seiner hinteren Position, der Ausgangsposition vor dem Einstechen der Nadel, gegen ein unbeabsichtigtes Vorschieben blockiert wird.

In einer besonders bevorzugten Ausführungsvariante wird das gleiche Sperrelement, das, wie vorstehend beschrieben, bereits zum Blockieren einer verschiebbaren Nadelschutzhülse verwendet wird, auch zum Blockieren des Vorschiebens des Behältnisses benutzt, d.h. das gleiche Sperrelement wird wahlweise für die beiden genannten Blockierfunktionen verwendet.

Der Abstreifer, die Blockierung der Nadelschutzhülse und die Blockierung des Behältnisses können mit Vorteil in Verbindung mit der kontrollierten Dämpfung eingesetzt werden, sie können jedoch auch jeweils für sich sowie in geeigneter Kombination untereinander bei einer Vorrichtung nach dem Oberbegriff von Anspruch 1 verwendet werden.

Bevorzugte Ausführungsbeispiele der Erfindung werden nachfolgend anhand von Figuren erläutert. Es zeigen:
- Fig. 1: ein erstes Ausführungsbeispiel einer erfindungsgemäßen Vorrichtung,
- Fig. 2a: den Abstreifer nach Figur 1 in einer hinteren Position,
- Fig. 2b: den Abstreifer nach Figur 1 in einer vorderen Position,
- Fig. 3: ein zweites Ausführungsbeispiel einer erfindungsgemäßen Vorrichtung,
- Fig. 4a: ein Autoinjektionsgerät mit erfindungsgemäßer Blockierung der Nadelschutzhülse,
- Fig. 4b: das Autoinjektionsgerät nach Figur 4a mit erfindungsgemäßer Blockierung des Behältnisses,
- Fig. 5: das Autoinjektionsgerät nach den Figuren 4a und 4b mit einem alternativen Auslösemittel und
- Fig. 6a-c: ein Autoinjektionsgerät mit ebenfalls erfindungsgemäßer Blockierung der Nadelschutzhülse.

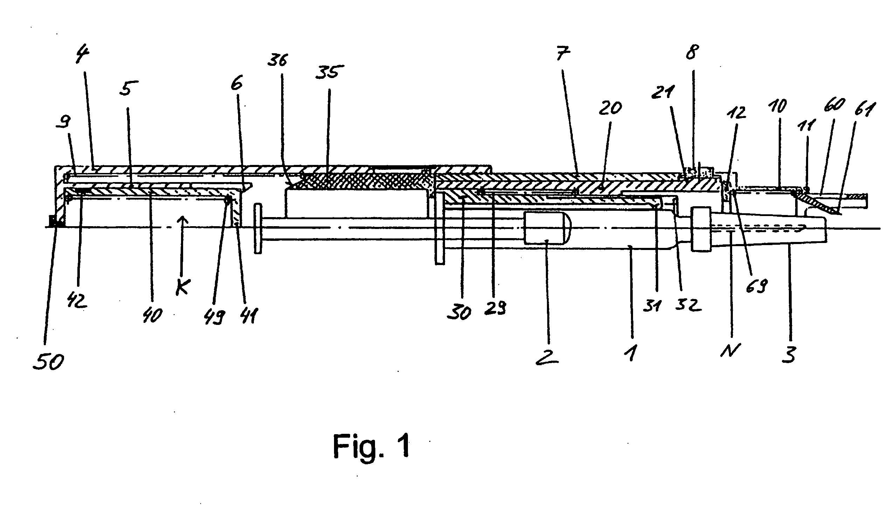

Fig. 1 zeigt im Längsschnitt einen Injektionspen mit einem langgestreckten, hohlzylindrischen Gehäuse als Basisteil, das eine hintere Gehäusehülse 4 mit einem Innengewinde in einem vorderen Bereich und eine vordere Gehäusehülse 7 mit einem Außengewinde in einem hinteren Bereich aufweist. Im Bereich der beiden Gewinde sind die beiden Gehäusehülsen 4 und 7 miteinander verschraubt. Im Bereich der vorderen Gehäusehülse 7 ist ein Behältnis 1 aufgenommen, das mit einem injizierbaren Produkt, insbesondere einer Medikamentflüssigkeit gefüllt ist.

Das Behältnis 1 ist eine für solche Injektionspens übliche Ampulle mit einem darin verschiebbar aufgenommenen Kolben 2. Durch Vorschieben des Kolbens 2 in Richtung auf einen Auslaß an einem vorderen Ende des Behältnisses 1 wird eine Produktdosis aus dem Behältnis 1 verdrängt. Am Behältnisauslaß ist eine in Vorschubrichtung des Kolbens 2 weisende Injektionsnadel N angebracht, die von einer Nadelschutzkappe 3 abgedeckt ist.

Das Behältnis 1 ist in einem Behältnishalter 30 aufgenommen und zentriert. Der Behältnishalter 30 wird ebenfalls durch eine Hülse gebildet, die in einem vorderen Bereich drei Zentrierlaschen 31 zum Zentrieren des Behältnisses 1 aufweist. Über den Umfang des Behältnishalters 30 in dessen vorderen Bereich gleichmäßig verteilt sind zwischen den Zentrierlaschen 31 über deren vorderen Enden hinausstehende weitere Laschen 32 gebildet, die als Anschlag beim Zurückschieben einer Nadelschutzhülse 10 dienen.

In einem Rinspalt zwischen dem Behältnishalter 30 und der vorderen Gehäusehülse 7 ist eine Zwischenhülse 20 angeordnet. Die Zwischenhülse 20 ist in ihrem hinteren Umfangsbereich mit Ausnehmungen versehen, die vom Behältnishalter 30 durchragt werden, der so mit der vorderen Gehäusehülse 7 verschiebesicher verbunden ist; im Ausführungsbeispiel ist der Behältnishalter 30 mit der vorderen Gehäusehülse 7 verschraubt. Die Zwischenhülse 20 hingegen ist relativ zur vorderen Gehäusehälfte 7 und dem Behältnishalter 30 nach Lösen einer Sperre 21 entgegen der Vorschubrichtung des Kolbens verschiebbar.

In der hinteren Gehäusehülse 4 ist eine Auslösehülse 35 angeordnet, die mit einer vorderen Stirnfläche an einer hinteren Stirnfläche der Zwischenhülse 20 anliegt. Die Auslösehülse 35 ist im wesentlichen einfach hohlzylindrisch ausgebildet und wird von einer vom Kolben 2 nach hinten wegragenden Kolbenstange durchragt. Sie ist in der hinteren Gehäusehülse 4 in Längsrichtung hin- und herverschiebbar aufgenommen. Von einer Rückstellfeder 9 wird sie in ihre in Figur 1 dargestellte vordere Position gedrückt, in der sie plan an einer hinteren Stirnfläche der Zwischenhülse 20 und einer hinteren Flanschfläche des Behältnisses 1 anliegt. Eine hintere Stirnfläche 36 der Auslösehülse 30 fällt von einer in einer inneren Mantelfläche umlaufenden, hinteren Kante schräg nach außen ab. Mittels der derart gebildeten Schrägfläche 36 wird bei einem Zurückschieben der Auslösehülse 35 eine Antriebseinrichtung zum Vorschieben des Kolbens 2 und Ausschütten des Produkts ausgelöst.

Die Antriebseinrichtung wird durch ein Abtriebsglied 40 und eine zwischen dem Abtriebsglied 40 und der hinteren Gehäusehülse 4 eingespannte, als Antriebselement 49 dienende Antriebsfeder gebildet. Das Abtriebsglied 40 ist topfförmig mit einem nach hinten offenen Hülsenkörper, gebildet durch eine einfach kreiszylindrische Hülse, die von einem geschlossenen Hülsenboden 41 nach hinten abragt. Dieser Hülsenkörper sitzt in einer ihn umgebenden Innenhülse 5 der hinteren Gehäusehülse 4, die von einer hinteren Stirnwand der hinteren Gehäusehülse 4 nach vom abragt. Die Innenhülse 5 bildet eine Gleitführung für das Abtriebsglied 40. Ferner hält sie mit mehreren gleichmäßig über ihren vorderen Umfangsbereich verteilt angeordneten, eine vordere Stirnfläche des Abtriebsglieds 40 umgreifenden Schnappelementen 6 das Abtriebsglied 40 gegen die Kraft des Antriebselements 49.

Figur 1 zeigt das Injektionsgerät in seiner Ausgangsstellung vor einer Injektion in der das Abtriebsglied 40 mittels der Schnappelemente 6 blockiert wird und die Nadel N von der Nadelschutzhülse 10 umgeben ist. Indem die Nadel N von der Nadelschutzhülse 10 umgeben wird, ist die Nadel insbesondere auch beim Einstechen in die Haut für einen Verwender des Injektionsgeräts verdeckt. Dies reduziert eine psychologische Hemmung, sich die Nadel N selbst einzustechen.

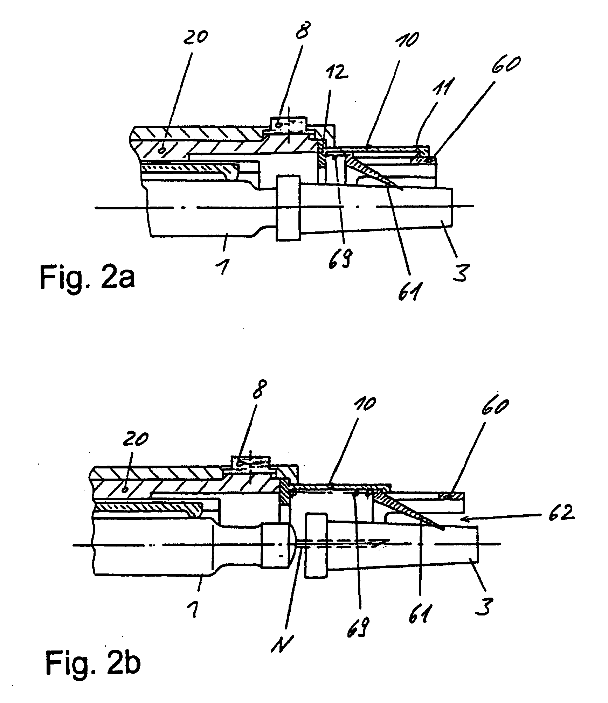

Zum Einstechen ist die Nadelschutzhülse 10 zusammen mit der Zwischenhülse 20 und der Auslösehülse 35 den Gehäusehülsen 4 und 7 sowie dem Behältnishalter 30 mit dem Behältnis 1 gegenüber entgegen der Vorschubrichtung der Nadel N zurückschiebbar. In der Nadelschutzhülse 10 ist ferner ein hülsenförmiger Abstreifer 60 gelagert. Der Abstreifer 60 ragt über eine vordere Stirnfläche der Nadelschutzhülse 10 vor und ist gegen ein in der Nadelschutzhülse 10 aufgenommenes Rückstellelement 69, das im Ausführungsbeispiel durch eine Rückstellfeder gebildet wird, in die Nadelschutzhülse 10 einschiebbar. Der Abstreifer 60 dient zum Abstreifen einer in Figur 1 bereits entfernten Nadelschutzkappe und wird später in Bezug auf die Figuren 2a und 2b in seiner Abstreiffunktion beschrieben.

Zum Injizieren des Produkts wird das Injektionsgerät auf die Oberfläche eines Gewebes, im allgemeinen die menschliche Haut, aufgesetzt und gegen das Gewebe gedrückt. Durch den Andruck wird zunächst der Abstreifer 60, der das vordere Ende des Injektionsgerätes bildet, gegen die Kraft des Rückstellelements 69 in die Nadelschutzhülse 10 bis in eine Anschlagstellung eingeschoben, in der er gänzlich oder nahezu gänzlich von der Nadelschutzhülse 10 aufgenommen wird. Sobald der Abstreifer 60 seine Anschlagstellung in der Nadelschutzhülse 10 erreicht hat, löst der Verwender die Sperre 21 durch Drücken eines Auslöseknopfs 8. Hierdurch wird die Verschiebeblockierung der Zwischenhülse 20 gegenüber der vorderen Gehäusehülse 7 gelöst.

Da das Injektionsgerät nach wie vor gegen das Gewebe gedrückt wird, werden jetzt die Nadelschutzhülse 10 und unter deren Andruck die Zwischenhülse 20 und infolgedessen die Auslösehülse 35 im Gehäuse nach hinten verschoben. Die Nadel N dringt in das Gewebe ein. Bevor die Nadelschutzhülse 10 mit einer hinteren Anschlagfläche, gebildet durch eine im hinteren Bereich der Nadelschutzhülse 10 radial nach innen ragenden Umlaufschulter 12, gegen die als Anschlag dienenden Laschen 32 des Behältnishalters 30 anschlägt, kommt die Schrägfläche 36 der Auslösehülse 35 in Eingriff mit den Schnappelementen 6 und löst beim weiteren Zurückschieben der Hülsen 10, 20 und 35 die Fixierung des Abtriebsglieds 40 in dem Moment, in dem die Nadel N die gewünschte, vorgegebene Eindringtiefe erreicht hat.

In diesem Moment wird das Abtriebsglied 40 durch die Antriebskraft des Antriebselements 49 in Vorschubrichtung vorgeschoben, gelangt in Andruck gegen eine hintere Stirnfläche der Kolbenstange und schiebt bei ihrem eigenen weiteren Vorschieben den Kolben 2 im Behältnis vor. Durch das Vorschieben des Kolbens 2 wird das Produkt aus dem Behältnis durch dessen Auslaß und die daran anschließende Nadel hindurch ausgeschüttet. Im Ausführungsbeispiel wird bei einem Auslösen der Antriebseinrichtung der gesamte Behältnisinhalt ausgeschüttet. Der Behältnisinhalt ist im Ausführungsbeispiel die Produktdosis. Grundsätzlich könnten jedoch auch durch konstruktive Fortbildung des Injektionsgeräts mehrere, vorgebbare Produktdosen erst bei mehreren Injektionen ausgeschüttet werden.

Beim Vorschieben des Abtriebsglieds 40 wird die im Antriebselement gespeicherte Antriebskraft allmählich, bei Verwendung einer Antriebsfeder als Antriebselement 49 beispielsweise entsprechend der Federcharakteristik, aufgezehrt. Die Vorschubgeschwindigkeit des Kolbens 2 würde daher im Verlaufe des Vorschiebens abnehmen und die Ausschüttrate sinken. Um das Nachlassen der Antriebskraft im Verlaufe des Vorschiebens des Kolbens 2 zu kompensieren, wird eine auf das Antriebsglied 40 wirkende pneumatische Dämpfungskraft erzeugt.

Zu diesem Zweck bilden das Antriebsglied 40 und das Gehäuse, im Ausführungsbeispiel die hintere Gehäusehülse 4, Wandungen einer Unterdruckkammer K, deren Volumen sich beim Vorschieben des Abtriebsglieds 40 vergrößert. Im Ausführungsbeispiel wird die Kammer K durch den Hülsenboden 41 und den davon abragenden Hülsenkörper des Abtriebsglieds 40 einerseits und die hintere Stirnfläche der hinteren Gehäusehülse 4 und die davon abragende Innenhülse 5 andererseits gebildet. Der Hülsenkörper des Abtriebsglieds 40 und die Innenhülse 5 werden relativ zueinander wie Teleskophülsen verschoben. Im Bereich der Gleitflächen, d.h. zwischen der äußeren Mantelfläche des Abtriebsglieds 40 und der Innenmantelfläche der Innenhülse 5 ist ein umlaufender Dichtkörper 42 angeordnet, im Ausführungsbeispiel sitzt ein Dichtring in einer Umfangsnut des Hülsenkörpers des Abtriebsglieds 40.

Die hintere Stirnfläche der hinteren Gehäusehülse 4 weist eine Durchgangsöffnung auf, in die ein Dichtkörper 50 mit einer kalibrierten Durchgangsbohrung, ansonsten jedoch luftdicht eingesetzt ist. Statt einer kalibrierten einfachen Durchgangsbohrung könnte beispielsweise auch ein Rückschlagventil verwendet werden, das ein ungehindertes Entweichen der Luft bei einem Zurückschieben des Abtriebsglieds 40 erlaubt, beim Einsaugen jedoch gegen einen Ventilsitz gepreßt, lediglich eine konstruktiv vorgegebene, definierte, enge Durchlaßöffnung freiläßt. Der pro Zeiteinheit in die Kammer K eingelassene Volumenstrom ist auf jeden Fall geringer als die pro Zeiteinheit mit dem Vorschieben des Abtriebsglieds 40 einhergehende Volumenvergrößerung der Kammer, so daß stets eine Dämpfungskraft erzeugt wird, solange das Abtriebsglied 40 durch eine resultierende Antriebskraft vorgeschoben wird. Die Dämpfungskraft ist umso größer, je schneller das Abtriebsglied 40 vorgeschoben wird, d.h. je größer die effektiv wirkende Antriebskraft ist. Durch die Art der Dämpfungskrafterzeugung wird somit automatisch ein in der Antriebseinrichtung stattfindener Energieverzehr kompensiert, da mit abnehmender Antriebskraft auch eine Verlangsamung der Vorschubbewegung des Antriebsglieds 40 und damit eine Verringerung der Dämpfungskraft einhergeht. Gleichzeitig werden jedoch auch Ungleichmäßigkeiten in allen weiteren, nicht vermeidbaren Gegenkräften mitkompensiert, da solche Unregelmäßigkeiten zu Verzögerungen oder Beschleunigungen des Abtriebsglieds 40 und damit automatisch zu Änderungen der vom Antriebselement 49 zu leistenden Volumenänderungsarbeit einhergehen. Solche Gegenkräfte sind beispielsweise die Wandreibung zwischen dem Behältnis 1 und dem Kolben 2, die über den Vorschubweg des Kolbens 2 im Behältnis 1 nicht überall gleich ist. Die Dämpfung nimmt wegen der Kompressibilität des Mediums und der Vergrößerung des Kammervolumens im Verlaufe des Vorschiebens des Abtriebsglieds 40 noch zusätzlich ab, so daß der Energieverzehr gleich zweifach kompensiert wird.

Aus den Figuren 2a und 2b ist ersichtlich, wie mit Hilfe des Abstreifers 60 eine Nadelschutzkappe 3 entfernt werden kann. In Figur 2a ist die Nadelschutzkappe 3 vollständig über die Injektionsnadel gestülpt. Dies entspricht dem Zustand des Injektionsgeräts unmittelbar nach dem Einsetzen des Behältnisses 1 und dem Zusammenschrauben der beiden Gehäusehülsen 4 und 7. Gleichzeitig ist dies die Transportstellung des Injektionsgeräts bis kurz vor einer Injektion. Zur Vorbereitung einer Injektion wird zunächst die Nadelschutzkappe 3 entfernt.

Figur 2a zeigt das Einleiten des Abstreifens der Nadelschutzkappe 3. Hierfür wird zunächst der hülsenförmige Abstreifer 60 gegen den Druck des Rückstellelements 69 in die Nadelschutzhülse 10 hineingeschoben bis er mit zwei einander diametral gegenüberliegend angeordneten Eingriffselementen 61 in Andruck gegen die Nadelschutzkappe 3 gelangt. Die beiden Eingriffselemente 61 ragen widerhakenartig von einer hinteren inneren Mantelfläche des Abstreifers 60 schräg nach innen vor. Beim Zurückschieben des Abstreifers 60 werden sie immer stärker gegen die sich nach hinten aufweitende Nadelschutzkappe 3 gepreßt.

Nachdem die Nadelschutzkappe 3 zwischen den Eingriffselementen 61 fest eingeklemmt ist, kann der Abstreifer 60 losgelassen werden. Er wird vom Rückstellelement 69 wieder in seine in Figur 2b dargestellte vordere Stellung gegen eine Anschlagschulter 11 der Nadelschutzhülse 10 geschoben und streift dabei die Nadelschutzkappe 3 vom Behältnis 1 ab. In dieser Stellung kann die lediglich noch lose über die Nadel N gestülpte Nadelschutzkappe 3 leicht per Hand nach vom gänzlich abgezogen werden. Um das Entfernen per Hand zu erleichtern, ist der Abstreifer 60 mit wenigstens zwei durchgreifbaren Ausnehmungen 62 versehen.

Indem der Abstreifer 60 am Injektionsgerät ständig befestigt ist, muß der Benutzer ihn nicht erst umständlich zum Abziehen der Nadelschutzkappe 3 einführen. Andererseits stört er bei der Injektion selbst in keiner Weise. Ein weiterer Vorteil ist darin zu sehen, daß die Nadelschutzhülse 10 vollkommen geschlossen, d.h. ohne Durchgriffschlitz zum Abstreifen der Nadelschutzkappe 3, ausgebildet sein und dadurch die Nadel N völlig verdecken kann.

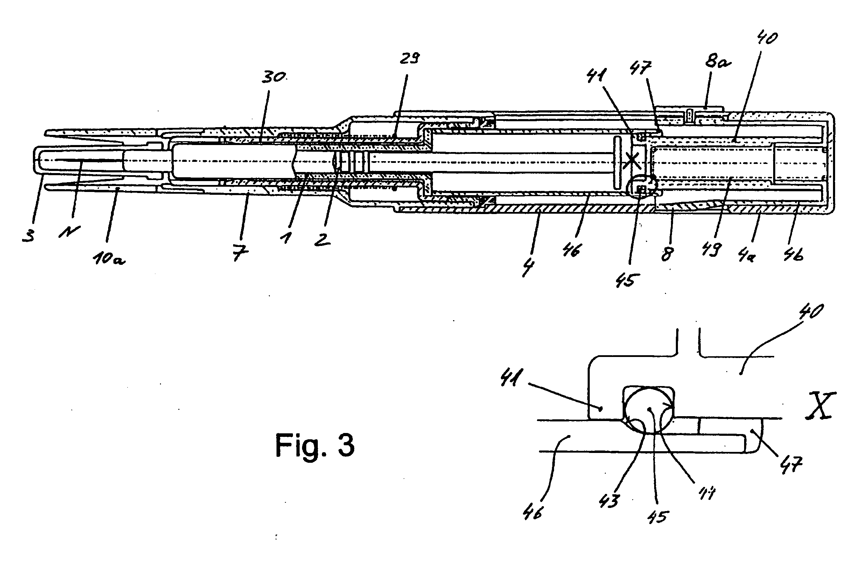

Figur 3 zeigt ein Autoinjektionsgerät, mit dem nicht nur die Ausschüttung des Produkts, sondern auch das Einstechen der Nadel automatisch erfolgt. Soweit in Figur 3 die gleichen Bezugszeichen wie im ersten Ausführungsbeispiel verwendet werden, handelt es sich um Komponenten gleicher Funktion. Was die grundsätzliche Funktionsweise des Autoinjektionsgerätes anbetrifft, so wird hiermit auf die parallele deutsche Patentanmeldung der Anmelderin vom gleichen Tag mit dem Titel "Autoinjektionsgerät" verwiesen.

Im Gegensatz zum Ausführungsbeispiel der Figur 1 erfolgt eine Dämpfung der auf das Abtriebsglied 40 wirkenden Antriebskraft beim Autoinjektionsgerät der Figur 3 mittels mechanischer Reibung. Die dämpfende Reibungskraft wirkt dabei zwischen einem als nachgiebiger Ring ausgebildeten Andruckelement 45, das zwischen dem hülsenförmigen Abtriebsglied 40 und einem beim Vorschieben das Abtriebsglied 40 umgebenden, ebenfalls hülsenförmigen Übertragungsglied 46 eingeklemmt ist.

Der Ablauf einer Injektion ist bei dem Autoinjektionsgerät der Figur 3 im wesentlichen der folgende: Durch Eindrücken einer Auslöselasche 8 wird eine die Vorschubbewegung des Abtriebsglieds 40 blockierende Sperre gelöst, und das Abtriebsglied 40 wird von der Antriebskraft des Antriebselements 49, das auch in diesem Ausführungsbeispiel eine Druckfeder ist, nach vorn, in Figur 3 nach links, geschoben. Zunächst bildet das Andruckelement 45 eine Kopplung zwischen dem Abtriebsglied 40 und dem Übertragungsglied 46, wie insbesondere auch in der Detaildarstellung X erkennbar ist. Über diese Kopplung nimmt das Abtriebsglied 40 das Übertragungsglied 46 mit, das seinerseits das Behältnis 1 mit der daran am vorderen Ende befestigten Nadel N dem Gehäuse gegenüber vorschiebt.

Hierdurch wird die Nadel N aus der in diesem Ausführungsbeispiel gehäusefesten Nadelschutzhülse 10a vorgestoßen und eingestochen. Das Einstechen wird durch Anschlagen des Behältnishalters 30 am Gehäuse begrenzt. Beim Anschlagen wird die Kopplung zwischen dem Abtriebsglied 40 und dem Übertragungsglied 46 gelöst, wie sich aus der Darstellung des Details X ohne weiteres ergibt. Bei seinem weiteren Vorschieben wird das Abtriebsglied 40 nun relativ zum Übertragungsglied 46 vorgeschoben, gelangt in Andruck gegen die Kolbenstange und schiebt bei seinem weiteren Vorschieben den Kolben 2 im Behältnis 1 vor, so daß das Produkt ausgeschüttet wird.

Das Andruckelement 45 ist als geschlitzter Federring in der Art eines Kolbenrings ausgebildet. Dieser Ring ist in einer an einem äußeren Umfang des Abtriebsglieds 40 umlaufenden Nut aufgenommen und drückt elastisch gegen eine innere Mantelgleitfläche des Übertragungsglieds 46. Indem diese innere Mantelgleitfläche in Vorschubrichtung sich erweiternd ausgebildet ist, nimmt die zwischen dem Andruckelement 45 und dem Übertragungsglied 46 wirkende Wandreibungskraft im Verlaufe des Vorschiebens das Abtriebsglieds 40 ab. Hierdurch wird das Nachlassen der Antriebskraft des Antriebselements 49 kompensiert.

In einem hinteren Bereich des Übertragungsglieds 46 ist ein Führungsring 47 aufgesetzt, der als Geradführung für das Abtriebsglied 40 dient. Dieser Führungsring 47 könnte auch als Dichtring mit einer oder mehreren kalibrierten Durchgangsbohrungen oder einem Rückschlagventil entsprechend dem Ausführungsbeispiel der Figur 1 ausgebildet sein. Auf diese Weise könnte statt der Reibungsdämpfungskraft oder auch zusätzlich dazu eine pneumatische Dämpfungskraft erzeugt werden. Die Unterdruckkammer würde bei solcher Ausbildung im Spaltbereich zwischen dem Übertragungsglied 46 und dem einschiebenden Antriebsglied 40 gebildet werden.

Mittels eines das Gehäuse durchragenden Spanngriffs 8a werden eine verschiebbar im Gehäuse aufgenommene Halte- und Auslösehülse 4b und damit zusammen das Abtriebsglied 10, die entsprechend miteinander verbunden sind, wieder in ihre hintere Stellung gebracht bzw. gespannt.

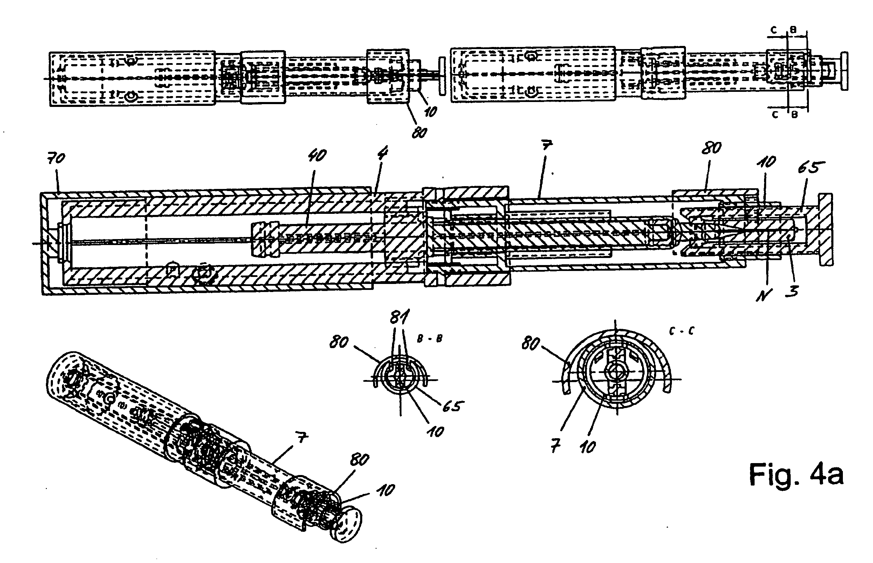

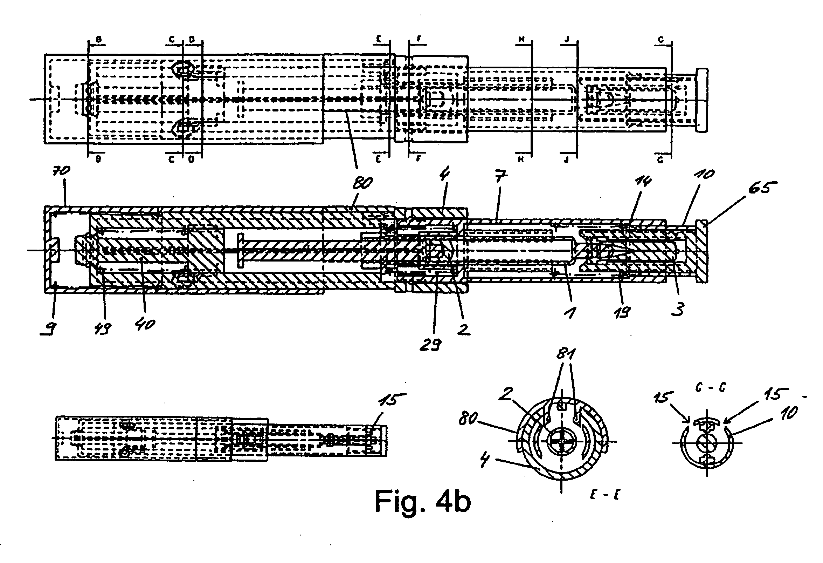

Die Figuren 4a und 4b zeigen ein weiteres Autoinjektionsgerät, bei dem im Gegensatz zum Autoinjektionsgerät der Figur 3 der Vorschub des Behältnisses 1 zum Einstechen der Nadel in herkömmlicher Weise über den Kolben 2, erfolgt, d.h. der Vorschub des Behältnisses 1 ist vom Vorschub des Kolbens 2 nicht entkoppelt, sondern erfolgt gerade über den Kolben 2. Das Injektionsgerät der Figuren 4a und 4b weist jedoch wie das der Figur 1 eine dem Gehäuse gegenüber hin- und herverschiebbare Nadelschutzhülse 10 auf. Diese verschiebbare Nadelschutzhülse 10 verdeckt, was beim Autoinjektionsgerät der Figur 3 nicht möglich ist, die Nadel nach der Injektion, d.h. nach dem Herausziehen.

Im Gegensatz zum Ausführungsbeispiel der Figur 1 ist jedoch ein Abstreifer 65 vorgesehen, der mit dem Injektionsgerät nicht ständig verbunden ist, sondern zum Abstreifen der Nadelschutzkappe 3 zwischen die Nadelschutzkappe 3 und die Nadelschutzhülse 10 eingeschoben werden muß, bis er die Nadelschutzkappe 3 klauenartig hintergreift, so daß sie mit dem Abstreifer 65 zusammen entfernt werden kann.

Figur 4a zeigt das Injektionsgerät nach einer Injektion nach dem Herausziehen der Nadel und bereits wieder aufgesetzter Nadelschutzkappe 3 für den weiteren Transport. Das Besondere dieses Ausführungsbeispiels ist eine Blockierung der Nadelschutzhülse 10.

Durch die Blockierung der Nadelschutzhülse 10 ist sicher gewährleistet, daß die Nadelspitze nicht frei vorstehen kann, wodurch die Gefahr von Beschädigungen der Nadel N und insbesondere die Gefahr von Verletzungen beseitigt wird. Die Nadelschutzhülse 10 wird durch ein Sperrelement 80 dem Gehäuse gegenüber blockiert, derart, daß die Nadelschutzhülse 10 nicht entgegen der Vorschubrichtung der Nadel N beim Einstechen zurückgeschoben werden kann.

Das Sperrelement 80 wird durch einen Teilring gebildet, der zwei Eingriffselemente 81 in Form von zwei Stegen aufweist, die von einer inneren Ringmantelfläche abragen. Die Nadelschutzhülse 10 weist, wie in Figur 4b links unten am besten zu erkennen ist, zwei Schlitze 15 auf, die von je einem der Eingriffselemente 81 durchgriffen werden, wenn das Sperrelement 80 auf das Gehäuse aufgesetzt wird. Die Eingriffselemente 81 bilden dann Anschläge für die in Umfangsrichtung sich erstreckenden Schlitzwandungen der Nadelschutzhülse 10. Das Sperrelement 80 wird durch Aufschneiden einer Hülse erhalten, im Ausführungsbeispiel einer Kreisringhülse, wobei das Aufschneiden in Längsrichtung und außerhalb der Mittellängsachse der Hülse erfolgt, so daß das Sperrelement 80 einen Schalenkörper aufweist, der im Querschnitt gesehen, ein Stück über den Halbkreis hinausragt und auf diese Weise, wie anhand der Figur 4b erläutert werden soll, über die hintere Gehäusehülse 4 aufsteckbar und deren Umfang ein Stück über den größten Durchmesser hinaus umgreifend gehalten ist.

In seiner Funktion als Sperre für die Nadelschutzhülse 10 wird das Sperrelement 80, wie am besten aus den Querschnitten B-B und C-C der Figur 4a zu erkennen ist, mittels Rastverbindung an der Nadelschutzhülse 10 gehalten. Hierfür sind die Eingriffselemente 81 elastisch nachgiebig ausgebildet und mit vorderen Rastnasen versehen, die nach Durchgriff des jeweiligen Schlitzes 15 eine der inneren Schlitzkanten umgreifen und das Sperrelement 80 wie Schnapper in seiner Sperrstellung halten, aber dennoch leicht wieder abgezogen werden können.

Figur 4b zeigt das Sperrelement 80 in seiner zweiten Funktion, in der es das Behältnis 1 gegen ein Vorschieben blockiert. Beim Abziehen der Nadelschutzkappe 3 würde das Behältnis 1 ohne die Blockierung ein Stück weit gegen die Kraft des elastischen Rückstellelements 29 nach vorne mitgezogen werden und im Moment des Abstreifens der Nadelschutzkappe 3 durch die Kraft des Rückstellelements 49 wieder in seine in Figur 4b dargestellte hintere Position zurückschnappen. Bei einem Zurückschnappen würde die Gefahr einer Beschädigung des Behältnisses und auch die daraus sich ergebende Gefahr eines eventuellen Blockierens der Vorschubbewegung des Behältnisses 1 beim Einstechen der Nadel N bestehen.

Um dies zu verhindern, wird das Sperrelement 80 in der Transportstellung des Injektionsgeräts bis nach dem Abziehen der Nadelschutzkappe 3 in einem den hinteren Rand des Behältnisses 1 überdeckenden Bereich des Gehäuses aufgesteckt. In dieser im Längsschnitt in Figur 4b dargestellten Stellung umgreift der Schalenkörper des Sperrelements 80, wie insbesondere im Querschnitt E-E zu erkennen ist, das Gehäuse eng und wird wegen seiner über den Halbkreis hinausragenden Enden an der hinteren Gehäusehülse 4 gehalten. Die Eingriffselemente 81 übernehmen keine Haltefunktion für das Sperrelement 80, sondern dienen nun der Blockierung des Behältnisses 1. Hierfür durchgreifen sie das Gehäuse und kommen im aufgesteckten Zustand des Sperrelements 80 vor einen am hinteren Ende des Behältnisses 1 umlaufenden Flansch zu liegen. Da das Behältnis 1 mit seinem hinteren, radial nach außen abragenden Flansch bei einem Abziehen der Nadelschutzkappe 3 auf die Eingriffselemente 81 des Sperrelements 80 drückt, ist das Behältnis 1 in seiner hinteren Position blockiert und kann daher nicht nach vorn gezogen werden.

Das Sperrelement 80 erfüllt in der in Figur 4b gezeigten Stellung eine weitere, dritte Funktion, da es ein Auslösen der Antriebseinrichtung verhindert, einfach indem es die Bewegung eines Auslösemittels 70 blockiert. Das Auslösemittel 70 kann das Abtriebsglied 40 dann aus seiner Blockierung lösen, wenn das Sperrelement 80 vom Gehäuse abgenommen und somit ein Vorschieben des Auslöseelements 70 dem Gehäuse gegenüber erst möglich ist.

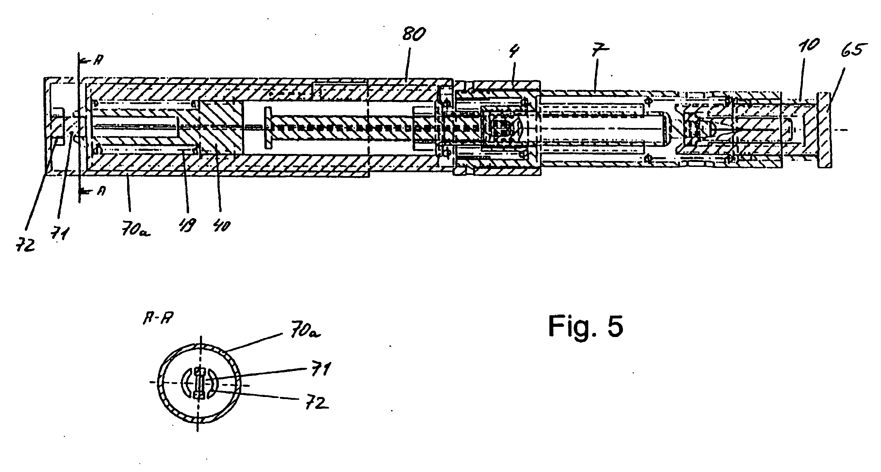

Figur 5 zeigt ein Autoinjektionsgerät, das mit Ausnahme des Auslösemittels 70 für das Abtriebsglied 40 dem Injektionsgerät der Figuren 4a und 4b entspricht. Ein Auslösemittel 70a der Figur 5 besteht wieder aus einem Hülsenkörper mit einem rückwärtigen Hülsenboden. Das Abtriebsglied 40 wird vom Auslösemittel 70a sowohl in seiner hinteren Position blockiert als auch nach entsprechender Betätigung des Auslösemittel 70a gelöst.

Das Abtriebsglied 40 läuft zu seinem rückwärtigen Ende hin in Schnappern aus, die durch eine hintere Gehäusestirnwand ragen und deren hinteren Umfangsrand umgreifen, wobei die Schnapper des Abtriebsglieds 40 infolge ihrer eigenen Elastizität nach außen gedrückt und bereits so gehalten werden. Als zusätzliche Sicherheit gegen unbeabsichtiges Entrasten, beispielsweise bei Stößen, ragt das Auslösemittel 70a mit einem von seinem Hülsenboden aufragenden Blockierstück 71, das im Querschnitt gesehen, eine schmale Reckteckform aufweist und mit seiner langen Seite zwischen zwei diametral gegenüber angeordneten Schnappern des Abtriebsglieds 40 eingreift. Es verhindert in dieser Drehstellung, daß die beiden Schnapper sich in der in Figur 5 gezeigten Stellung des Auslösemittels 70a aufeinander zu biegen können und so die Rastverbindung gelöst würde. Ferner ragen vom Hülsenboden des Auslösemittels 70a beidseits vom Blockierstück 71 beabstandet zwei Andruckstücke 72 ab, die im Querschnitt die Form von Teilkreissegmente haben. Das Blockierstück 71 überragt die Andruckstücke 72. Zum Auslösen des Abtriebsglieds 40 wird zunächst das Sperrelement 80 entfernt, dann wird das Auslösemittel 70a zumindest soweit um seine Längsachse verdreht, daß die Schnapper des Abtriebsglieds 40 aufeinander zu gebogen werden können. Mit dem Verdrehen des Auslösemittels 70a gelangen die Andruckstücke 72 im Querschnitt gesehen über die Schnapper des Abtriebsglieds 40. Beim Drücken des Auslösemittels 70a in Richtung auf das Abtriebsglied 40 zu drücken die Andruckstücke 42 gegen die Schnapper des Abtriebsglieds 40, die rückwärtige Schrägflächen aufweisen und so unter dem Andruck der Andruckstücke 72 aufeinander zu gebogen werden und die Rastverbindung des Abtriebsglieds 40 mit dem Gehäuse gelöst wird. Unter dem Andruck des Antriebselements 49 wird dann das Abtriebsglied 40 vorgeschoben.

Das Sperrelement 80 kann statt als Steckelement auch als Drehelement ausgebildet sein und könnte dadurch insbesondere am Gehäuse verbleiben, nachdem die Blockierung des Behältnisses gelöst worden ist.

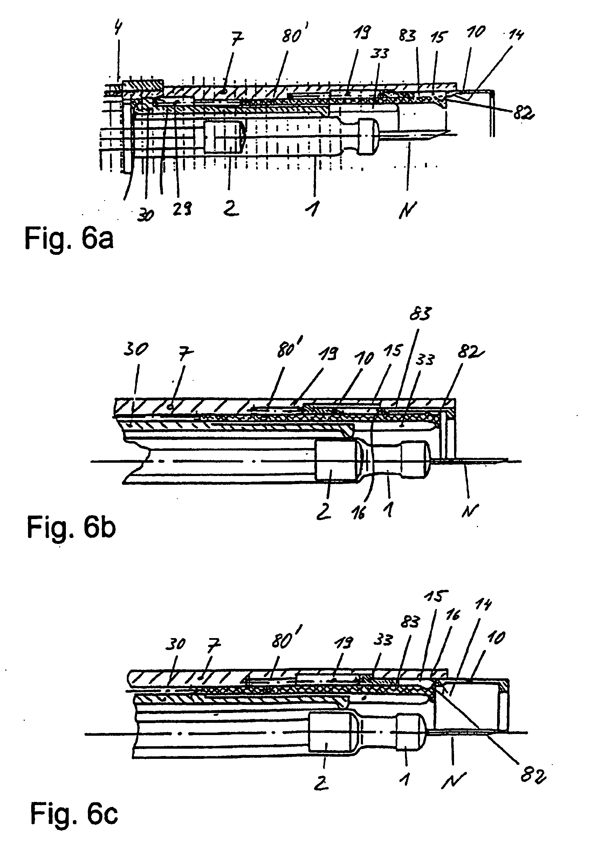

Die Figuren 6a, 6b und 6c zeigen die Wirkungsweise der Nadelschutzvorrichtung, die verhindert, daß die Nadel N nach dem Herausziehen aus dem Gewebe frei aus dem Gehäuse hervorsteht und abgebrochen werden sowie Verletzungen bei unachtsamer Handhabung verursachen kann. Das wesentliche Merkmal der Nadelschutzvorrichtung ist, daß die zum Zwecke des Einstechens dem Gehäuse gegenüber verschiebbare Nadelschutzhülse 10 nach dem Herausziehen der Nadel in einer Nadelschutzstellung blockiert ist, so daß sie nicht mehr ins Gehäuse geschoben werden kann; ein außenseitiges Überschieben des Gehäuses wäre ebenso denkbar. Die Funktionsweise des Pens zum Einstechen der Nadel N und zum Ausschütten des Produkts entspricht der des Pens nach Fig. 3.

Fig. 6a zeigt den vorderen Teil des Pen in seiner Ausgangsstellung unmittelbar vor der Injektion. Fig. 6b zeigt den Pen in der vorderen Position des Behältnisses B, d.h. in der Injektionsstellung. Die Nadelschutzhülse 10 ist gegen die Kraft des Rückstellelements 19 in ihre hinterste Verschiebeposition der Gehäusehülse 7 gegenüber gedrückt worden. Die Nadel N ragt um die gewünschte Länge aus dem Gehäuse und der Nadelschutzhülse 10 vor.

Die Nadelschutzhülse 10 weist eine hintere Anschlagfläche und eine vordere Anschlagfläche auf, die den Verschiebeweg der Nadelschutzhülse 10 gegenüber der vorderen Gehäusehülse 7 in Vorschubrichtung und gegen die Vorschubrichtung begrenzen. Beim Hin- und Herschieben fährt die Nadelschutzhülse 10 über eine im Gehäuse verschiebegesichert und vorzugsweise auch verdrehgesichert aufgenommene Sperrhülse 80', die an ihrem vorderen Ende wenigstens einen schräg oder bogenförmig nach außen abragenden Haken 82 aufweist. Die Nadelschutzhülse 10 weist in einem Innenmantelbereich, mit dem sie über den Haken 82 gleitet, der Länge ihres maximalen Verschiebewegs in etwa entsprechend, eine Verbreiterung, vorzugsweise einen leicht verbreiterten Innendurchmesser, auf. Ein Übergangsbereich 14 von der Verbreiterung zum anschließenden Innenquerschnitt ist abgeschrägt, so daß die Nadelschutzhülse 10 unter dem Andruck des Rückstellelements 19 bis hinter den Übergangsbereich 14 über den Haken 82 gleiten kann. Die Nadelschutzhülse 10 ist hinter dem Übergangsbereich 14 in einem mittleren Bereich mit Längsschlitzen 15 versehen, deren vordere Stirnflächen 16, wie am besten in Fig. 6c zu erkennen ist, Anschlagflächen für je einen der Haken 82 bilden.

Die Sperrhülse 80' läuft in mehreren gleichmäßig über den Hülsenumfang verteilt geordneten elastisch nachgiebigen Zungen 83 aus, an deren freien vorderen Enden je einer der Haken 82 ausgebildet sind. Der Behältnishalter 30 läuft zu seinem freien vorderen Ende hin ebenfalls in Zungen 33 aus. Durch Vorschieben des Behältnishalters 30 gegen die Kraft des Rückstellelements 29 kommen dessen Zungen 33 unter die Zungen 83 der Sperrhülse 80' zu liegen. Jede der Zungen 83 wird so radial nach innen abgestützt und kann in der vorderen Behältnisposition nicht mehr radial einwärts gebogen werden. Die Zungen 83 werden von den Zungen 33 nicht nur abgestützt, sondern zusätzlich radial nach außen gedrückt. Die Zungen 33 sind verglichen mit den Zungen 83 starr ausgebildet oder können in erster Näherung diesen Zungen 83 gegenüber als starr angenommen werden.

Nach dem Herausziehen der Nadel N wird die Nadelschutzhülse 10 durch das Rückstellelement 19 wieder nach vorn geschoben. Sie überschiebt wegen des schrägen Verlaufs der Fläche 14 und/oder des entsprechenden Schrägverlaufs des wenigstens einen Hakens 82 diesen Haken 82, dessen auslaufende Spitze zudem elastisch nachgiebig ist. Sobald jedoch die Nadelschutzhülse 10 soweit wieder vorgeschoben worden ist, daß ihre Anschlagfläche 16 in Vorschubrichtung gesehen vor den Haken 82 zu liegen kommt, wird sie durch den gegen die Anschlagfläche 16 auf Anschlag liegenden Haken 82 gegen ein Zurückschieben gesperrt. Der Haken 82 und die Nadelschutzhülse 10 liegen mit senkrecht zur Verschieberichtung weisenden Anschlagflächen aneinander. Die Nadel N wird in der in Figur 6c gezeigten Sicherheitsstellung nach der Injektion durch die Nadelschutzhülse 10 geschützt.

Der Behältnishalter 30 wird somit gleichzeitig auch als verschiebbare Unterstützung für das wenigstens eine elastische Sperrmittel 82 genutzt und erfüllt nach der Erfindung die Doppolfunktion des Halterns des Behältnisses 1 und des Sperrens der Nadelschutzhülse 10. Die Nadelschutzvorrichtung setzt nicht die erfindungsgemäße Ausbildung des Autoinjektionsgeräts voraus, obgleich sie besonders bevorzugt in Kombination damit Verwendung findet. Sie ist ebenso mit Vorteil bei gattungsgemäßen Autoinjektionsgeräten verwendbar.

## Patentansprüche

1. Vorrichtung zur Verabreichung eines injizierbaren Produkts, wenigstens umfassend:
a) ein Basisteil (4, 7),
b) ein vom Basisteil (4, 7) aufgenommenes Behältnis (1), aus dem durch Verschieben eines darin aufgenommenen Kolbens (2) in eine Vorschubrichtung eine Produktdosis durch eine Nadel (N) hindurch ausgeschüttet wird, und
c) eine Antriebseinrichtung wenigstens mit einem Abtriebsglied (40) und einem Antriebselement (49), das auf das Abtriebsglied (40) bei Betätigung der Antriebseinrichtung eine Antriebskraft ausübt, durch die das Abtriebsglied (40) in Vorschubrichtung des Kolbens (2) verschoben wird und dabei den Kolben (2) im Behältnis (1) vorschiebt, d
d) **dadurch gekennzeichnet, daß** zum Abstreifen einer über die Nadel (N) gesteckten Nadelschutzkappe (3) ein Abstreifer (60) an der Vorrichtung in Längsrichtung der Nadel (N) hin- und herverschiebbar angebracht ist, derart, dass der Abstreifer (60) bei einer Injektion an der Vorrichtung verbleiben kann und zum Einstechen der Nadel (N) zurückgeschoben wird.

2. Vorrichtung nach Anspruch 1, **dadurch gekennzeichnet, dass** der Abstreifer (60) mit Eingriffsmitteln (61) zum Klemmen oder Greifen der Nadelschutzkappe (3) versehen ist.

3. Vorrichtung nach dem vorhergehenden Anspruch, **dadurch gekennzeichnet, dass** die Eingriffsmittel (61) einander diametral gegenüberliegend angeordnet sind und widerhakenartig von einer hinteren inneren Mantelfläche des Abstreifers (60) schräg nach innen vorragen.

4. Vorrichtung nach einem der zwei vorhergehenden Ansprüche, **dadurch gekennzeichnet, dass** eine Nadelschutzhülse (10) aus einer Ausgangsstellung, in der sie die Nadel (N) bis über die Nadelspitze umgibt, in eine Verabreichungsstellung, in der die Nadel (N) aus der Nadelschutzhülse (10) vorragt, relativ zum Basisteil (4, 7) verschiebbar angeordnet ist und dass die Nadelschutzhülse (10) gegen ein Verschieben in Richtung auf die Verabreichungsstellung dem Basisteil (4, 7) gegenüber blockierbar ist.

5. Vorrichtung nach dem vorhergehenden Anspruch, **dadurch gekennzeichnet, dass** der Abstreifer (60) in der Nadelschutzhülse (10) gelagert ist.

6. Vorrichtung nach dem vorhergehenden Anspruch, **dadurch gekennzeichnet, dass** der Abstreifer (60) gegen ein Rückstellelement (69) in die Nadelschutzhülse (10) einschiebbar ist.

7. Vorrichtung nach einem der vorhergehenden Ansprüche, **dadurch gekennzeichnet, dass** die Vorrichtung ein Autoinjektionsgerät ist, dass das Behältnis (1) mit der daran angebrachten Nadel (N) zum Einstechen der Nadel (N) relativ zum Basisteil (4, 7) aus einer Ausgangsstellung in eine Verabreichungsstellung vorschiebbar ist und dass das Behältnis (1) in der Ausgangsstellung durch lösbaren Eingriff eines Sperrmittels (80; 82) gegen ein Vorschieben blockiert wird.

8. Vorrichtung nach Anspruch 4 und 7, **dadurch gekennzeichnet, dass** das Sperrmittel (80) wahlweise entweder das Behältnis (1) gegen Vorschieben oder die Nadelschutzhülse (10) gegen Zurückschieben blockiert.

9. Vorrichtung nach einem der vorhergehenden Ansprüche, **dadurch gekennzeichnet, dass** Mittel (42, 50; 45, 46) vorgesehen sind, die eine Dämpfungskraft erzeugen, die der Antriebskraft im Verlaufe eines Vorschiebens des Kolbens (2) zusätzlich zu allen nicht vermeidbaren Gegenkräften entgegenwirkt.

10. Vorrichtung nach dem vorhergehenden Anspruch, **dadurch gekennzeichnet, dass** die Dämpfungskraft im Verlaufe des Verschiebens des Kolbens abnimmt.

11. Vorrichtung nach Anspruch 9 oder 10, **dadurch gekennzeichnet, dass** das Abtriebsglied (40) wenigstens eine Wandung einer Kammer (K) bildet, die wenigstens eine Kammeröffnung für ein in die Kammer (K) einströmendes oder aus der Kammer (K) ausströmendes Medium aufweist, wobei die Kammeröffnung einen Druckausgleich bei einem Vorschieben des Abtriebsglieds (40) und einer damit einhergehenden Volumenänderung der Kammer (K) nur verzögert zulässt.

12. Vorrichtung nach dem vorhergehenden Anspruch, **dadurch gekennzeichnet, dass** die Kammer (K) eine Unterdruckkammer ist.

13. Vorrichtung nach Anspruch 9 oder 10, **dadurch gekennzeichnet, dass** zur Erzeugung der Dämpfungskraft ein Andruckelement (45) vorgesehen ist, das eine Klemmkraft zwischen dem Abtriebsglied (40) und einem Gegenelement (46) überträgt, wobei das Abtriebsglied (40) oder das Gegenelement (46) eine Andruckfläche für das Andruckmittel (45) aufweist, die sich in Vorschubrichtung des Abtriebsglieds (40) derart erstreckt, dass die Klemmkraft im Verlaufe des Vorschiebens abnimmt.

14. Vorrichtung nach einem der vorhergehenden Ansprüche, **dadurch gekennzeichnet, dass** das Andruckelement (45) ein nachgiebiger Ring ist, der in einem zwischen dem Antriebsglied (40) und dem Gegenelement (46) gebildeten, sich im Verlaufe des Vorschiebens des Abtriebsglieds (40) aufweitenden Spalt angeordnet ist.

## Claims

1. A device for administering an injectable product, comprising at least:
a) a base section (4, 7);
b) a container (1) accommodated by the base section (4, 7) from which a product dose is dispensed through a needle (N) by displacing a piston (2), arranged in the container, in a forward direction; and
c) a drive unit comprising at least a driven member (40) and a drive element (49) which applies a drive force to the driven member (40) when the drive unit is actuated, by which drive force the driven member (40) is displaced in the forward direction of the piston (2), thus advancing the piston (2) within the container (1);
d) **characterised in that** a stripper (60) is arranged on the device, such that it can be moved back and forth in a longitudinal direction of the needle (N), in order to strip off a needle safety cap (3) applied to the needle (N), such that the stripper (60) can remain on the device during an injection and is pushed back in order to insert the needle (N).

2. The device according to claim 1, **characterised in that** the stripper (60) is provided with engaging means (61) for clamping or gripping the needle safety cap (3).

3. The device according to the preceding claim, **characterised in that** the engaging means (61) are arranged diametrically opposite each other and project obliquely inwards from a rear inner surface area of the stripper (60).

4. The device according to any one of the preceding two claims, **characterised in that** a needle safety sleeve (10) is arranged such that it can be displaced from a base position, in which it surrounds the needle (N) beyond the tip of the needle, to an administering position, in which the needle (N) projects out from the needle safety sleeve (10), in relation to the base section (4, 7), and **in that** the needle safety sleeve (10) can be blocked against being displaced towards the administering position in relation to the base section (4, 7).

5. The device according to the preceding claim, **characterised in that** the stripper (60) is mounted in the needle safety sleeve (10).

6. The device according to the preceding claim, **characterised in that** the stripper (60) can be inserted, against a restoring element (69), into the needle safety sleeve (10).

7. The device according to any one of the preceding claims, **characterised in that**: the device is an auto-injection device; the container (1), including the needle (N) attached to it, can be advanced from a base position to an administering position in relation to the base section (4, 7), in order to insert the needle (N); and **in that** in the base position, the container (1) is blocked against advancing by a releasable engagement of a blocking means (80; 82).

8. The device according to claims 4 and 7, **characterised in that** the blocking means (80) selectively blocks either the container (1) against advancing or the needle safety sleeve (10) against retracting.

9. The device according to any one of the preceding claims, **characterised in that** means (42, 50; 45, 46) are provided which generate a damping force which counteracts the drive force in the course of advancing the piston (2) in addition to any unavoidable counter forces.

10. The device according to the preceding claim, **characterised in that** the damping force decreases in the course of displacing the piston.

11. The device according to claim 9 or 10, **characterised in that** the driven member (40) forms at least one wall of a chamber (K) comprising at least one chamber port for a medium flowing into the chamber (K) or out of the chamber (K), wherein when the driven member (40) is advanced and there is an accompanying change in the volume of the chamber (K), the chamber port allows only for a delayed pressure compensation.

12. The device according to the preceding claim, **characterised in that** the chamber (K) is a low-pressure chamber.

13. The device according to claim 9 or 10, **characterised in that** a contact pressure element (45) is provided for generating the damping force and transmits a clamping force between the driven member (40) and a counter element (46), wherein the driven member (40) or the counter element (46) comprises a contact pressure surface for the contact pressure means (45), the contact pressure surface extending in a forward direction of the driven member (40), such that the clamping force decreases in the course of advancing.

14. The device according to any one of the preceding claims, **characterised in that** the contact pressure element (45) is a pliable ring arranged in a gap which is formed between the driven member (40) and the counter element (46) and widens in the course of advancing the driven member (40).

## Revendications

1. Dispositif d'administration d'un produit injectable, comprenant au moins :
a) un élément de base (4, 7),
b) un récipient (1) logé dans l'élément de base (4, 7) et à partir duquel, en déplaçant dans un sens d'avance un piston (2) qui y est logé, une dose de produit est déversée à travers une aiguille (N) et
c) un dispositif d'entraînement comprenant au moins un élément de sortie (40) et un élément d'entraînement (49) qui exerce sur l'élément de sortie (40), lorsque le dispositif d'entraînement est actionné, une force d'entraînement grâce à laquelle l'élément de sortie (40) est déplacé dans le sens d'avance du piston (2) en faisant avancer le piston (2) dans le récipient (1),
d) **caractérisé en ce que**, pour enlever un capuchon de protection d'aiguille (3) emboîté sur l'aiguille (N), un dispositif d'enlèvement (60) est placé sur le dispositif dans le sens longitudinal de l'aiguille (N) de manière à pouvoir être déplacé dans les deux sens, de telle manière que le dispositif d'enlèvement (60) puisse rester sur le dispositif pendant une injection et soit repoussé pour enfoncer l'aiguille (N).

2. Dispositif selon la revendication 1, **caractérisé en ce que** le dispositif d'enlèvement (60) est doté de moyens de prise (61) pour bloquer ou agripper le capuchon de protection d'aiguille (3).

3. Dispositif selon la revendication précédente, **caractérisé en ce que** les moyens de prise (61) sont disposés diamétralement opposés les uns par rapport aux autres et dépassent obliquement vers l'intérieur à partir d'une surface d'enveloppe arrière intérieure du dispositif d'enlèvement (60) à la manière d'ardillons.

4. Dispositif selon une des deux revendications précédentes, **caractérisé en ce qu'**une douille de protection d'aiguille (10) est disposée de manière à pouvoir être déplacée par rapport à l'élément de base (4, 7) depuis une position initiale dans laquelle elle entoure l'aiguille (N) jusqu'au-delà de la pointe de l'aiguille jusque dans une position d'administration dans laquelle l'aiguille (N) dépasse de la douille de protection d'aiguille (10) et **en ce que** la douille de protection d'aiguille (10) peut être bloquée par rapport à l'élément de base (4, 7) à l'encontre d'un déplacement en direction de la position d'administration.

5. Dispositif selon la revendication précédente, **caractérisé en ce que** le dispositif d'enlèvement (60) est logé dans la douille de protection d'aiguille (10).

6. Dispositif selon la revendication précédente, **caractérisé en ce que** le dispositif d'enlèvement (60) peut être inséré dans la douille de protection d'aiguille (10) contre un élément de rappel (69).

7. Dispositif selon une des revendications précédentes, **caractérisé en ce que** le dispositif est un appareil d'injection automatique, **en ce que** le récipient (1) avec l'aiguille (N) placée dessus peut être avancé depuis une position initiale jusque dans une position d'administration par rapport à l'élément de base (4, 7) pour enfoncer l'aiguille (N) et **en ce que** le récipient (1) est bloqué en position initiale à l'encontre d'un déplacement par prise amovible d'un moyen de blocage (80, 82).

8. Dispositif selon la revendication 4 ou 7, **caractérisé en ce que** le moyen de blocage (80) bloque au choix soit le récipient (1) contre une avance, soit la douille de protection d'aiguille (10) contre un recul.

9. Dispositif selon une des revendications précédentes, **caractérisé en ce que** sont prévus des moyens (42, 50 ; 45, 46) qui génèrent une force d'amortissement qui agit en sens contraire de la force d'entraînement pendant une avance du piston (2) en plus de toutes les forces antagonistes non évitables.

10. Dispositif selon la revendication précédente, **caractérisé en ce que** la force d'amortissement diminue pendant le déplacement du piston.

11. Dispositif selon la revendication 9 ou 10, **caractérisé en ce que** l'élément de sortie (40) forme au moins une paroi d'un compartiment (K) qui comporte au moins un orifice de compartiment pour un fluide qui afflue dans le compartiment (K) ou s'écoule hors du compartiment (K), l'orifice du compartiment ne permettant qu'avec un décalage une compensation de pression lors d'une avance de l'élément de sortie (40) et d'une modification de volume du compartiment (K) qui y est associée.

12. Dispositif selon la revendication précédente, **caractérisé en ce que** le compartiment (K) est un compartiment en dépression.

13. Dispositif selon la revendication 9 ou 10, **caractérisé en ce que**, pour générer la force d'amortissement, il est prévu un élément de pression (45) qui transmet une force de blocage entre l'élément de sortie (40) et un contre-élément (46), l'élément de sortie (40) ou le contre-élément (46) comportant pour le moyen de pression (45) une surface de pression qui s'étend dans le sens d'avance de l'élément de sortie (40) de telle manière que la force de blocage diminue pendant l'avance.

14. Dispositif selon une des revendications précédentes, **caractérisé en ce que** l'élément de pression (45) est un anneau souple qui est disposé dans une fente formée entre l'élément d'entraînement (40) et le contre-élément (46) et s'élargit pendant l'avance de l'élément de sortie (40).
